# EUROPEAN PATENT SPECIFICATION

(11) **EP 2 923 704 B1**
(45) Date of publication and mention of the grant of the patent: **20.03.2024**
(21) Application number: 13854909.2
(22) Date of filing: 15.11.2013
(51) Int. Cl.: A61K 35/74, A61P 1/04, A61P 1/12, A61P 25/02, C12N 1/20, C12R 1/245

(54) **STRESS-INDUCED BOWEL DISORDER-RELIEVING AGENT COMPRISING SPECIFIC LACTOBACILLUS GASSERI STRAIN OR TREATMENT PRODUCT THEREOF**
MITTEL ZUR LINDERUNG EINER STRESSBEDINGTEN DARMERKRANKUNG MIT EINEM SPEZIFISCHEN LACTOBACILLUS-GASSERI-STAMM ODER EINEM BEHANDLUNGSPRODUKT DARAUS
AGENT SOULAGEANT LES TROUBLES INTESTINAUX INDUITS PAR LE STRESS COMPRENANT UNE SOUCHE SPÉCIFIQUE DE LACTOBACILLUS GASSERI OU PRODUIT DE TRAITEMENT ASSOCIÉS;

(30) Priority: 16.11.2012 JP 2012252642; 16.11.2012 JP 2012252422; 16.11.2012 JP 2012252527; 22.01.2013 JP 2013009332
(43) Date of publication of application: 30.09.2015
(73) Proprietor: Asahi Group Holdings, Ltd., Tokyo 130-8602 (JP)
(72) Inventor: SAWADA Daisuke, Sagamihara-shi Kanagawa 252-0206 (JP); FUJIWARA Shigeru, Sagamihara-shi Kanagawa 252-0206 (JP)
(74) Representative: D Young & Co LLP
(86) International application number: PCT/JP2013/080930
(87) International publication number: WO 2014/077365

(56) References cited:
- WO-A1-2012/105312
- WO-A1-2013/084971
- JP-A- 2003 095 963
- JP-A- 2004 305 128
- JP-A- 2005 536 197
- JP-A- 2011 135 804
- JP-A- 2012 017 282
- JP-A- 2012 158 568
- JP-A- 2012 188 409
- VERMEIREN L ET AL: "Evaluation of meat born lactic acid bacteria as protective cultures for the biopreservation of cooked meat products", INTERNATIONAL JOURNAL OF FOOD MICROBIOLOGY, ELSEVIER BV, NL, vol. 96, no. 2, 1 November 2004 (2004-11-01), pages 149-164, XP004567738, ISSN: 0168-1605, DOI: 10.1016/J.IJFOODMICRO.2004.03.016
- TOSHIHIRO SASHIHARA ET AL: "Effect of growth conditions of Lactobacillus gasseri OLL2809 on the immunostimulatory activity for production of interleukin-12 (p70) by murine splenocytes", INTERNATIONAL JOURNAL OF FOOD MICROBIOLOGY, vol. 120, no. 3, 1 December 2007 (2007-12-01), pages 274-281, XP055013338, ISSN: 0168-1605, DOI: 10.1016/j.ijfoodmicro.2007.09.003
- DAISUKE SAWADA ET AL: 'CONTINUOUS ORAL INTAKE OF FERMENTED MILK PRODUCT WITH LACTOBACILLUS GASSERI STRAIN CP 2305:A POTENTIAL AND PRACTICAL APPROACH TO STRESS PROTECTION' JOURNAL OF INTESTINAL MICROBIOLOGY vol. 23, no. 2, 2009, page 96, XP008174116
- DAISUKE SAWADA ET AL: 'EFFECT OF LACTOBACILLUS GASSERI STRAIN CP2305 ON SYMPOMS OF CRF-INDUCED DIARRHEA AND STUDY ON ITS WORKING MECHANISM' JOURNAL OF INTESTINAL MICROBIOLOGY vol. 27, no. 2, April 2013, page 119, XP008179970

## Description

### Technical Field

The present invention relates to an agent for alleviating (or an agent for suppressing) excessive water secretion in the bowels of a subject, which comprises living cells or sterilized cells of a specific *Lactobacillus gasseri* strain or a treated product thereof, as an active ingredient, or an agent for alleviating (or an agent for suppressing) stress-induced bowel disorder, and particularly stress-induced diarrheal symptoms.

Further, described herein is a novel cold-tolerant *Lactobacillus gasseri* strain or a method for producing the same.

The present invention further relates to a stress-induced bowel disorder-alleviating agent (or stress-induced bowel disorder-suppressing agent) comprising the cold-tolerant *Lactobacillus gasseri* strain or a treated product thereof as an active ingredient.

Further described herein is a food additive or an animal feed additive, or, a food or a pharmaceutical composition, comprising the above alleviating agent (or the suppressing agent).

### Background Art

There is some Prior Art Literature demonstrating the stress-suppressing effect of a lactic acid bacterium of the genus *Lactobacillus* and the possible effectiveness thereof against gastrointestinal diseases, as exemplified as follows.

For example, JP Patent Publication No. 2009-102292 A (Patent Literature 1) describes a stress-suppressing agent containing a lactic acid bacterium FERM P-19169 (that is, *Lactobacillus paracasei* subsp. Paracasei) as an active ingredient, on the basis of an effect of regulating cytokine production, a prophylactic effect against an infection, a fattening effect, or an effect of suppressing diarrhea. The literature describes that stress suppression can trigger alleviation of the symptoms including hypophagia, dyspepsia, insomnia, physical deconditioning, and infectious diseases through the suppression of biological non-specific reactions caused by stressors that are external stimuli inducing stress.

JP Patent Publication No. 2009-100692 A (Patent Literature 2) and JP Patent Publication No. 2008-212140 A (Patent Literature 3) describe a lactic acid bacterium formulation for mammals which comprises a microorganism of the genus *Lactobacillus* having an effect of relieving stress. Specifically the literature describes that when a specific lactic acid bacterium is administered to a mammal, an excellent effect of relieving stress is exhibited and gastrointestinal diseases such as a stress-induced diarrheal disease can be prevented and treated.

JP Patent No. 4350170 B (Patent Literature 4) describes a pharmaceutical preparation required for preventing and/or treating gastrointestinal disorders in humans and animals, which comprises a survivable microbial strain of *Lactobacillus casei rhamnosus* LB21 having the accession No. NCIMS 40564 in at least one pharmaceutically acceptable carrier medium in which the microorganism can maintain its viability.

JP Patent Publication No. 2011-200211 A (Patent Literature 5) describes an anti-ulcer agent comprising *Lactobacillus gasseri* MCC1183 strain, which is used for preventing or treating gastric ulcer or duodenal ulcer caused by *Helicobacter pylori.*

JP Patent Publication No. 2009-511471 A (Patent Literature 6) describes use of at least 1 probiotic bacterial strain selected from lactic acid bacteria for the manufacture of a pharmaceutical composition for treating and/or preventing an autoimmune disease, wherein the autoimmune disease is selected from the group consisting of multiple sclerosis (MS), allergy, psoriasis, rheumatoid arthritis, Crohn's disease, ulcerative colitis, type I diabetes, inflammatory bowel disease, and organ specific autoimmunities including systemic lupus erythematosus; and the lactic acid bacterial strain is selected from the group consisting of *Lactobacillus plantarum, Lactobacillus rhamnosus, Lactobacillus fermentum, Lactobacillus* paracasei, and *Lactobacillus gasseri.*

JP Patent No. 4853986 B (Patent Literature 7) describes an agent for preventing and treating inflammatory bowel disease and/or irritable bowel syndrome comprising cells or a fermented product of *Lactobacillus gasseri* SBT2055 (referred to as "LG2055") (FERM P-15535) as an active ingredient. The literature also describes that in general treatment for irritable bowel syndrome, psychological stress is controlled and drug therapy is performed in stages in accordance with symptoms, with the administration of an intestinal remedy, a laxative, and then an antipsychotic agent.

Daisuke Sawada et al., the 13th annual meeting of Intestinal Microbiology (2009), Tokyo, Japan (Non-Patent Literature 1) describes that living cells of *Lactobacillus gasseri* CP2305 strain have an effect of alleviating stress, which improves a stress-induced abdominal pain, for example.

Daisuke Sawada at al., the 10th Symposium on Lactic Acid Bacteria (2011), the Netherlands, Federation of European Microbiological Societies (FEMS) and Netherlands Society for Microbiology (NVvM) (Non-Patent Literature 2) describe the administration of living cells of the CP2305 strain to irritable bowel syndrome (IBS) patients, but describe nothing about alleviation of diarrheal symptoms.

JP Patent Publication No. 2003-095963 A (Patent Literature 8) describes an agent for preventing and treating inflammatory bowel disease or irritable bowel syndrome with the use of cells or a fermented product of *Lactobacillus gasseri.*

### Prior Art Literature

### Patent Literature

Patent Literature 1: JP Patent Publication No. 2009-102292 A
Patent Literature 2: JP Patent Publication No. 2009-100692 A
Patent Literature 3: JP Patent Publication No. 2008-212140 A
Patent Literature 4: JP Patent No. 4350170 B
Patent Literature 5: JP Patent Publication No. 2011-200211 A
Patent Literature 6: JP Patent Publication No. 2009-511471 A
Patent Literature 7: JP Patent No. 4853986 B
Patent Literature 8: JP Patent Publication No. 2003-095963 A

### Non-Patent Literature

Non-Patent Literature 1; Daisuke Sawada et al., the 13th Annual Meeting of Intestinal Microbiology (2009), Tokyo, Japan
Non-Patent Literature 2: Daisuke Sawada et al., the 10th Symposium on Lactic Acid Bacteria (2011), the Netherlands, Federation of European Microbiological Societies (FEMS) and Netherlands Society for Microbiology (NVvM)

### Summary of the Invention

### Problems to Be Solved by the Invention

There has been described that a *Lactobacillus gasseri* strain that is a type of lactic acid bacterium is effective for preventing or treating gastrointestinal disorders such as inflammatory bowel disease and irritable bowel syndrome. Also described is that such gastrointestinal disorders are assumed to be related to stress. However, there is no report in the prior-art on a (cold-tolerant) lactic acid bacterium having an effect of alleviating stress-induced bowel disorder in cases in which a *Lactobacillus gasseri* strain exhibits an effect of suppressing excessive water secretion and significantly prevents, improves, or treats stress-induced diarrheal symptoms while suppressing gastrointestinal disorders (and, in particular, stress) and maintaining intestinal hypermotility at a normal level. Moreover, there is no report that a *Lactobacillus gasseri* strain significantly improves stress-induced diarrheal symptoms caused by exposure to stress in cases in which an intracerebral stress hormone is secreted while maintaining intestinal hypermotility at a normal level. In particular, no literature has described the suppression (alleviation or relief) of stress-induced diarrhea resulting from stress caused to healthy individuals that are differentiated from IBS patients. An object of the present invention is to use living cells or sterilized cells of *Lactobacillus gasseri* or a treated product thereof for the prevention and treatment of excessive water secretion or stress-induced disorders that occur in association with excessive water secretion, such as stress-induced diarrhea or stress-induced abdominal pain.

Furthermore, there is no known lactic acid bacterium having resistance to low temperatures that are problematic for production, and having an effect against stress-induced bowel disorder, for example.

### Means for Solving the Problems

In a first embodiment, the invention provides the Lactobacillus gasseri CP2305 strain (FERM BP-11331), a cold-tolerant derivative strain derived from Lactobacillus gasseri CP2305 strain (FERM BP-11331), a treated product thereof, or a mixture thereof for use in suppressing stress-induced diarrhea in a healthy subject, wherein the treated product thereof is dried cells, broken cells, disrupted cells, or in the form of fermented milk.

In a second embodiment, the invention provides the Lactobacillus gasseri CP2305s strain (NITE BP-1405) or a cold-tolerant derivative strain derived from Lactobacillus gasseri CP2305 strain (FERM BP-11331) or the CP2305s strain (NITE BP-1405), a treated product thereof, or a mixture thereof for use in alleviating excessive water secretion in the bowels, stress-induced diarrhea, stress-induced bowel disorder, irritable bowel syndrome, or inflammatory bowel disease, wherein the treated product thereof is dried cells, broken cells, disrupted cells, or in the form of fermented milk.

In a third embodiment, the invention provides a food or drink, preferably a functional food or drink, either:
a) comprising living cells or sterilized cells of a Lactobacillus gasseri strain according to embodiment 1, a treated product thereof, or a mixture thereof, for use according to embodiment 1; or
b) comprising living cells or sterilized cells of a Lactobacillus gasseri strain according to embodiment 2, a treated product thereof, or a mixture thereof, for use according to embodiment 2.

In a fourth embodiment, the invention provides a lactic acid bacterium, which is Lactobacillus gasseri CP2305s strain (NITE BP-1405), or a cold-tolerant derivative strain derived from Lactobacillus gasseri CP2305 strain (FERM BP-11331) or the CP2305s strain.

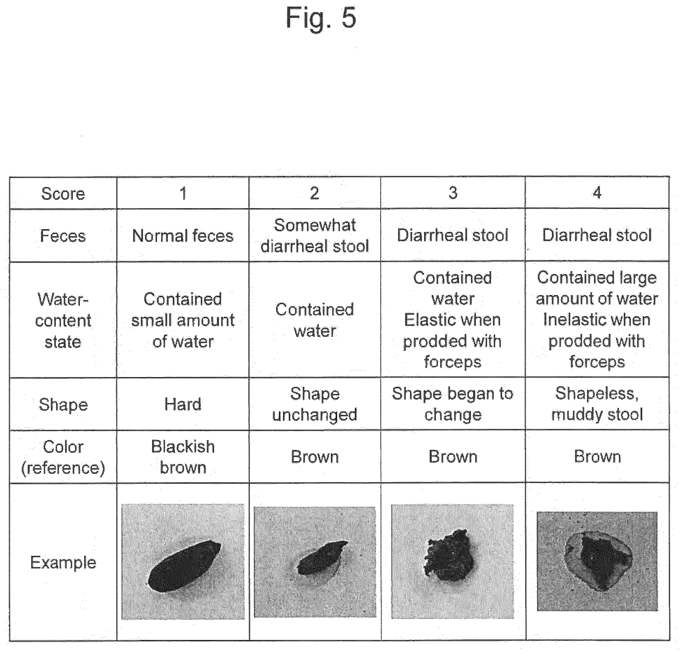

Here, the term "diarrhea score described in Example 3 described later" refers to, specifically, the diarrhea score evaluated by the method described in Example 3 herein. Briefly, the term refers to the sum total of the ten results of "fecal weight (value "i") × fecal score (value "ii")". The value "i" denotes the fecal weight (g) measured, and the value "ii" denotes the score determined on the basis of fecal score criteria (that is, score 1 = normal feces; score 2 = somewhat diarrheal stool; score 3 = shape began to change, diarrheal stool; and score 4 = shapeless diarrheal stool) as shown in Fig. 5.

Moreover, the term "the method described in Example 3 described later" refers to, briefly, a method that involves feeding a rat (4-week-old, male) ad libitum with a feed mixture containing test cells for 3 weeks, which has been prepared by mixing 25 g of CRF-1 (Trademark) feed with a lyophilized powder of 2.0 × 10¹⁰ test cells, administering intraperitoneally a corticotropin releasing factor (CRF) solution to the rat at 125 µg/kg to induce stress-induced diarrhea, evaluating the fecal status for 100 minutes after administration of CRF using the diarrhea score (described above) (19 rats per group), and then evaluating the suppression of stress-induced diarrhea by test cells based on the result.

*Lactobacillus gasseri* CP2305 strain (FERM BP-11331), *Lactobacillus gasseri* CP2305s strain (NITE BP-1405), or a cold-tolerant derivative strain derived from *Lactobacillus gasseri* CP2305 strain (FERM BP-11331) or a cold-tolerant derivative strain derived from the CP2305s strain, a treated product thereof, or a mixture thereof according to the present invention maintains intestinal motility at a normal level by inhibiting the stress-induced suppression of pelvic (parasympathetic) neural activity, as well as suppresses effectively the stress-induced diarrhea in a subject. Hence, they are effective for preventing, alleviating, or treating stress-induced bowel disorder. In particular, *Lactobacillus gasseri* CP2305s strain (NITE BP-1405) or a cold-tolerant strain derived from *Lactobacillus gasseri* CP2305 strain (FERM BP-11331) or the CP2305s strain, a treated product thereof, or a mixture thereof according to the present invention maintains intestinal motility at a normal level by inhibiting the stress-induced suppression of pelvic (parasympathetic) neural activity, significantly suppresses the excessive water secretion in the bowels of a subject, and effectively suppresses the stress-induced diarrhea in a subject, for example, and thus is effective for preventing, alleviating, or treating stress-induced bowel disorder.

### Brief Description of the Drawings

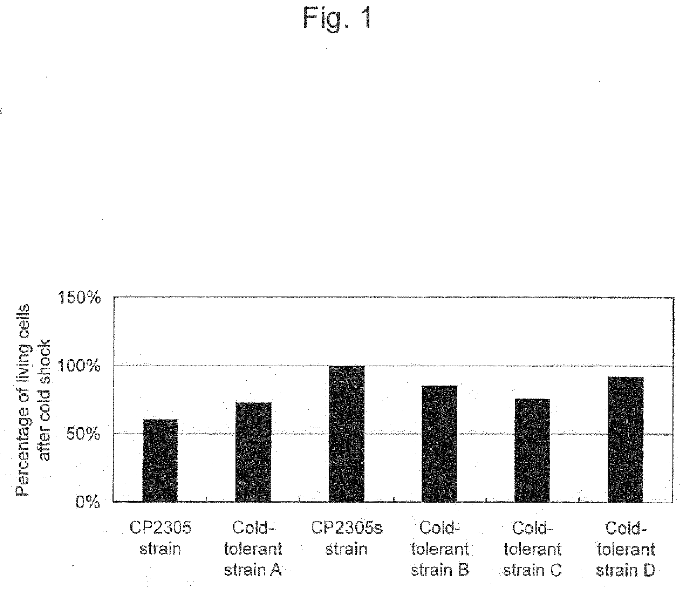

Fig. 1 shows the percentages of living cells of cold-tolerant strains (CP2305s, and A to D) obtained by subjecting *Lactobacillus gasseri* CP2305 strain to cold shock.

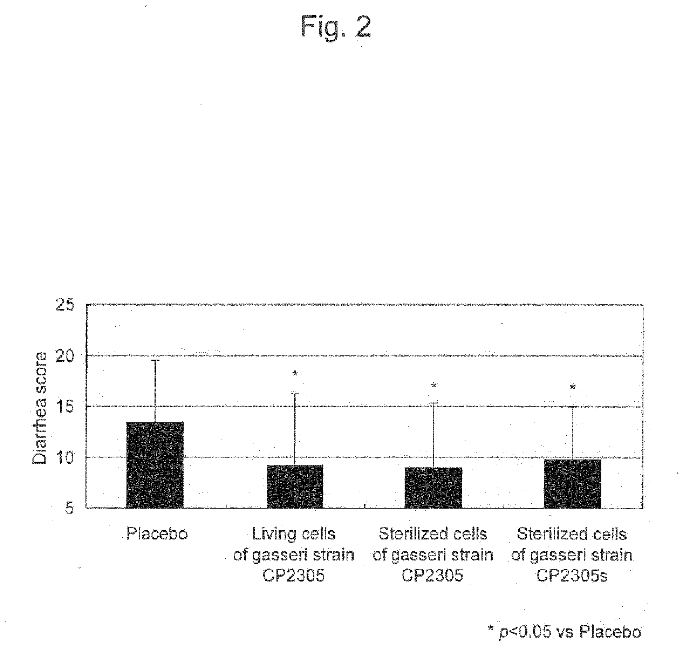

Fig. 2 shows the effects of living cells and sterilized cells of *Lactobacillus gasseri* CP2305 strain, and sterilized cells of the derivative strain CP2305s on a CRF-induced diarrhea model. A placebo is a negative control containing no CP2305 strain. In addition, "*" indicates that significance level "p" is less than 0.05.

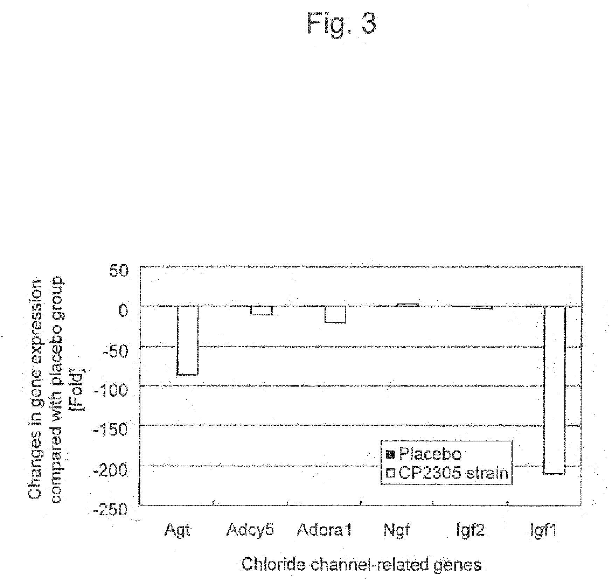

Fig. 3 shows changes in colon chloride channel-related gene expression after CRF induction. While in the placebo group, the expression levels of chloride channel-related genes were increased after administration of CRF, the expression levels of the same were suppressed in the group to which the CP2305 strain had been administered. In Fig. 3, "Agt" denotes angiotensinogen (serpin peptidase inhibitor, clade A, member 8), "Adcy5" denotes adenylate cyclase 5, "Adora1" denotes Adenosine receptor A1, "Ngf" denotes a nerve growth factor (beta polypeptide), "lgf2" denotes insulin-like growth factor 2, and "Igf1" denotes insulin-like growth factor 1.

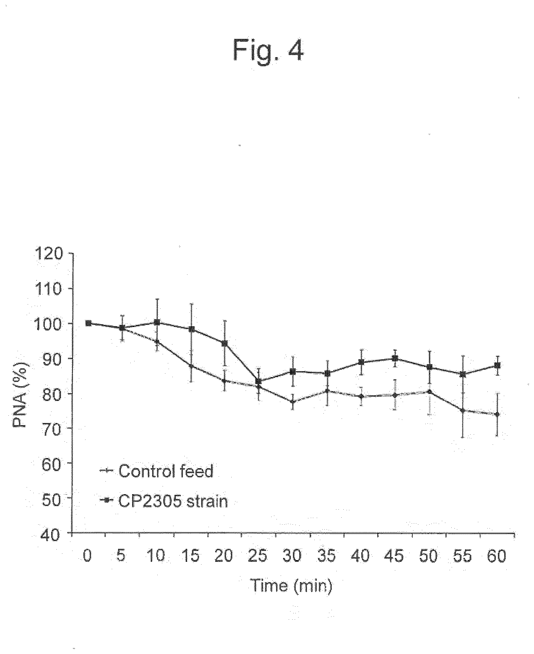

Fig. 4 shows the effects of the administration of the CP2305 strain on pelvic (parasympathetic) nerve.
Fig. 5 shows the fecal score criteria for calculating the diarrhea score in an experiment concerning the present invention. The fecal score was evaluated with 4 stages (1 to 4) based on the "water content state" and "shape" of feces (The color of feces in this table is not evaluated for the fecal score criteria, but is added herein as referential information showing the effect of the present invention).

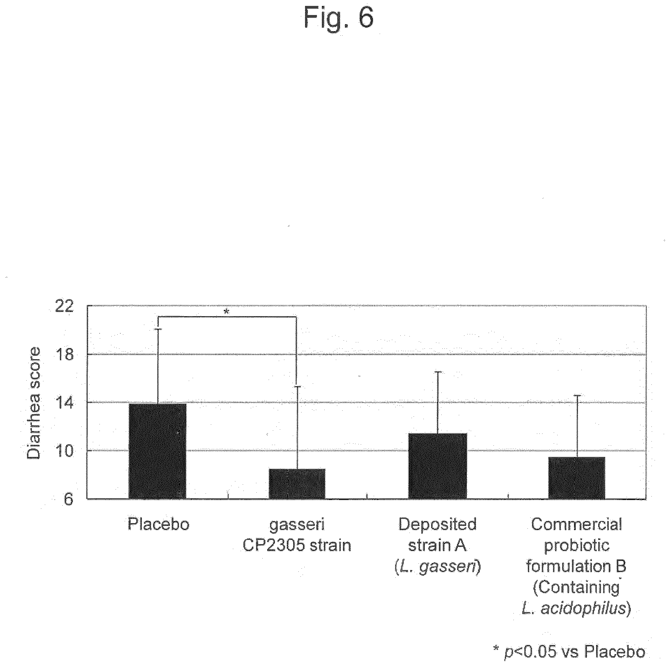

Fig. 6 shows the effects of the administration of various lactic acid bacteria on a CRF-induced diarrhea model. Lactic acid bacteria used herein are living cells of *Lactobacillus gasseri* CP2305 strain, known deposited strain A (L. *gasseri*)*,* and commercial probiotic formulation B (containing *L. acidophilus*)*.* The placebo is a negative control containing no CP2305 strain. In addition, "*" indicates that significance level "p" is less than 0.05.

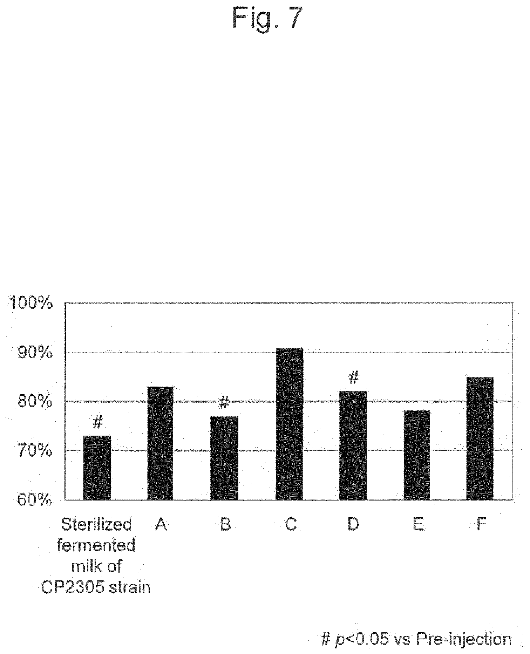

Fig. 7 shows the effects of the administration of sterilized fermented milk of various lactic acid bacteria on the nerve stimulation-induced ion transport in the large intestine. The vertical axis shows the percentages of ion secretion found when the same of a placebo (negative control) was expressed as 100%. In Fig. 7, A: known deposited strain A (*L*. *gasseri*)*,* B: *L. acidophilus,* C: *B. pseudocatenulatum,* D: *L. delbrueckii,* E: *S. thermophilus,* and F: *L. kefirgranum.* "#" indicates that significance level "p" is less than 0.05.

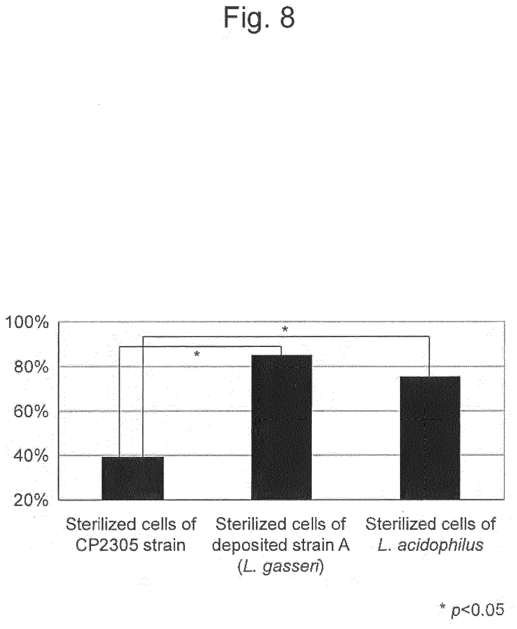

Fig. 8 shows the effects of the administration of sterilized cells of the CP2305 strain, known deposited strain A (*L. gasseri*)*,* and *L. acidophilus* on the nerve stimulation-induced ion transport in rat large intestine (distal colon). The vertical axis shows the percentages of ion secretion found when the same of a placebo (negative control) was expressed as 100%. "*" indicates that significance level "p" is less than 0.05.

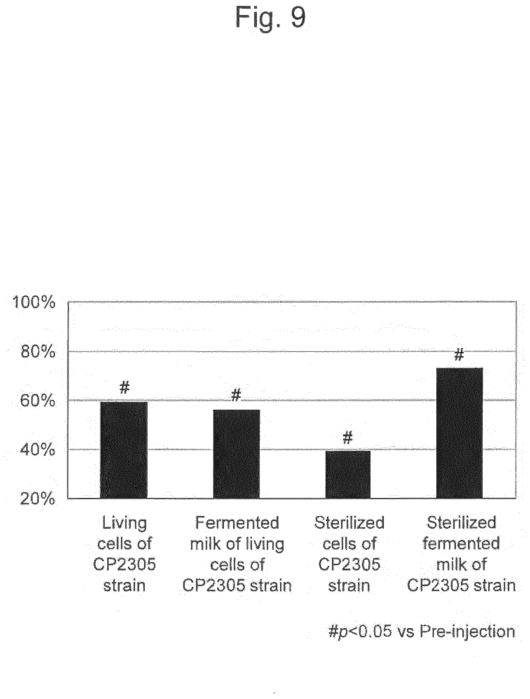

Fig. 9 shows the effects of the administration of living cells and sterilized cells of the CP2305 strain, and the fermented milk of living cells of and the sterilized fermented milk of the CP2305 strain on the ion transport in rat large intestine (distal colon). The vertical axis shows the percentages of ion secretion when the same of a placebo (negative control) was expressed as 100%. "#" indicates that significance level "p" is less than 0.05.

### Modes for Carrying out the Invention

The present invention will hereafter be described in greater detail.

### <Agent for suppressing excessive water secretion in the bowels>

Described herein is an agent for suppressing excessive water secretion in the bowels of a subject, comprising living cells or sterilized cells of *Lactobacillus gasseri* CP2305 strain (FERM BP-11331) (hereinafter, also simply referred to as "CP2305 strain"), a treated product thereof, or a mixture thereof, as an active ingredient.

### <Cold-tolerant Lactobacillus gasseri strain>

Further described herein is *Lactobacillus gasseri* CP2305s strain (NITE BP-1405) (hereinafter, also simply referred to as "CP2305s strain"), or a cold-tolerant derivative strain derived from *Lactobacillus gasseri* CP2305 strain (FERM BP-11331) or the CP2305s strain.

A cold-tolerant strain can be produced by a method that involves exposing a *Lactobacillus gasseri* strain to a temperature of 10°C or lower, selecting cold-tolerant strains that have survived, breeding the strains, optionally repeating the steps at least once, as necessary, and then collecting cold-tolerant strains that have survived. Through the procedure, after cold shock, a cold-tolerant strain with a percentage (of living cells) of about 60% or higher, preferably about 70% or higher, further preferably about 80% or higher, 85% or higher, and 90% or higher can be obtained.

With the use of the cold shock method, several cold-tolerant strains could be obtained (Fig. 1) including *Lactobacillus gasseri* CP2305s strain (NITE BP-1405). *Lactobacillus gasseri* CP2305s strain was internationally deposited by the applicant under NITE BP-1405 with the NITE (National Institute of Technology and Evaluation) Patent Microorganisms Depositary (NPMD) (2-5-8 Kazusakamatari, Kisarazu-shi, Chiba, 292-0818 Japan) as of August 14, 2012, which is an international depositary authority as stipulated by the Budapest Treaty.

In addition, *Lactobacillus gasseri* CP2305 strain that is a parent strain was internationally deposited by the applicant under FERM BP-11331 with the International Patent Organism Depositary, the National Institute of Advanced Industrial Science and Technology (AIST) (Tsukuba Central 6, 1-1-1 Higashi, Tsukuba, Ibaraki, 305-8566, Japan) as of September 11, 2007, which is an international depositary authority as stipulated by the Budapest Treaty. This strain has been transferred to and is currently stored in an international depositary authority, the NITE (National Institute of Technology and Evaluation) Patent Microorganisms Depositary (NPMD) (2-5-8 Kazusakamatari, Kisarazu-shi, Chiba, 292-0818 Japan).

### <Agent for alleviating stress-induced bowel disorder, etc.>

The present invention further provides an agent for alleviating excessive water secretion in the bowels, stress-induced diarrhea, stress-induced bowel disorder, irritable bowel syndrome, diarrhea-predominant irritable bowel syndrome or inflammatory bowel disease, which comprises *Lactobacillus gasseri* CP2305 strain (FERM BP-11331), *Lactobacillus gasseri* CP2305s strain (NITE BP-1405) or a cold-tolerant derivative strain derived from *Lactobacillus gasseri* CP2305 strain (FERM BP-11331) or the CP2305s strain, a treated product thereof, or a mixture thereof, as an active ingredient.

Cells of the CP2305 strain, the CP2305s strain, or a cold-tolerant derivative strain derived from the CP2305 strain or the CP2305s strain may be either living cells or sterilized cells (dead cells).

The term "subject" as used herein include animals belonging to mammals and birds, and preferably primates such as humans, livestock animals such as cattle, horses, sheep, goats, pigs, chickens, and turkeys, racing animals such as horses, and pet animals such as dogs and cats.

in a manner similar to that for the CP2305 strain or the CP2305s strain, other derivative strains from the CP2305 strain or the CP2305s strain also have an effect of alleviating stress-induced bowel disorder to a degree equivalent to or higher than that of its parent strain and an effect of suppressing excessive water secretion in the bowels, and, can be obtained as lactic acid bacteria belonging to the species *Lactobacillus gasseri* differing from the above strains. Such a derivative strain can be prepared by placing a parent strain under unusual conditions for the parent strain, such as exposing a parent strain to ultraviolet light, culturing a parent strain in the presence of a mutagenic substance, culturing a parent strain at a temperature other than the optimum temperature, culturing a parent strain in the presence of an organic solvent, or the like, Examples of a mutagenic substance include 3-amino-1,4-dimethyl-5H-pyrido[4,3-b)indole (Trp-P-1), 2-amino-6-methytimida-5H- pyrido[1,2-a-3',2'-d]-imidazale (Glu-P-1), 2-amino-3,4-dimethylimidazo [4,5-f]quinoline (MeIQ), 4-nitroquinoline-N-oxide (4NQO), and N-methyl-N'-nitro-N-nitrosoguanidine (MNNG). A target derivative strain can be selected by, for example, using as indicators (1) having an effect of alleviating stress-induced bowel disorder to a degree equivalent to or higher than that of *Lactobacillus gasseri* CP2305 strain (FERM BP-11331) or *Lactobacillus gasseri* CP2305s strain (NITE BP-1405), such that its effect of alleviating stress-induced bowel disorder is an effect of suppressing stress-induced diarrhea to a degree equivalent to or higher than that of the above strain in terms of diarrhea score, (2) having a survival rate of 60% or higher when the bacteria are kept on ice for 5 or more hours, (3) having an effect of inhibiting the stress-induced suppression of pelvic (parasympathetic) neural activity, and (4) having an effect of suppressing water secretion by suppressing the chloride channels of the large intestine. The present invention also relates to lactic acid bacteria having such property of (1), and any one of the properties of (2) to (4).

Regarding known related *Lactobacillus gasseri* strains, *Lactobacillus gasseri* SBT 2055 strain (FERM P-15535) having prophylactic and therapeutic effects on inflammatory bowel disease or irritable bowel syndrome has been known (JP Patent Publication No. 2003-095963 A). This strain is a lactic acid bacterial strain isolated and cultured from the intestine of Japanese subjects, and has been reported to suppress blood in stool and melena more effectively than a control group in a test using DDS-induced ulcerative colitis model and living cells. Moreover, *Lactobacillus gasseri* OLL2716 strain (FERM BP-6999) (JP Patent Publication No. 2004-305128A) that improves intestinal flora to reduce the onset of diarrhea has been known. The document describes in examples that the incidence of diarrhea within 2 weeks after the introduction of the strain to the experimental farm was decreased during the maintenance of calves. However, these known *Lactobacillus gasseri* strains are, as described below, clearly different from the CP2305 strain, and its cold-tolerant derivative strain, the CP2305s strain.

Living cells and sterilized cells of the CP2305s strain that is a cold-tolerant strain of the CP2305 strain, or a cold-tolerant derivative strain from the CP2305 strain or the CP2305s strain, as well as treated products of these strains thereof according to the present invention have unexpectedly an effect of suppressing excessive water secretion from the bowels, and an effect of significantly suppressing stress-induced diarrhea (see Fig. 1 and Fig. 2). These effects are especially significant, even when the sterilized fermented milk of a parent strain, *Lactobacillus gasseri* CP2305 strain, is compared with the sterilized fermented milk of known deposited *Lactobacillus gasseri* strains, *L. acidophilus, B. pseudocatenulatum, L. delbrueckii, S. thermophiles,* and *L. kefirgranum* (Fig. 7). As a result of thorough analysis of gene expression in the colon tissues of rats to which CRF had been administered, it was demonstrated that excessive water secretion from the bowels is suppressed because the CP2305 strain suppresses the chloride channels of the large intestine, so as to control the transfer of water (see Fig. 3). It was demonstrated this time that the CP2305 strain also has an effect of inhibiting the stress-induced suppression of pelvic (parasympathetic) neural activity by measuring the electrical activity of pelvic (parasympathetic) nerve that innervates the lower part of the large intestine. The administration of the CP2305 strain restores the bowel functions of a subject induced by stress at a normal level and maintains the normal level. Accordingly, the CP2305 strain has a special effect of significantly alleviating diarrheal symptoms of stress-induced bowel disorder. The above CP2305s strain and a derivative strain thereof such as a cold-tolerant strain also have an effect of alleviating stress-induced bowel disorder, and particularly stress-induced diarrhea.

*Lactobacillus gasseri* CP2305 strain, *Lactobacillus gasseri* CP2305s strain, or a cold-tolerant derivative strain from the CP2305 strain or the CP2305s strain according to the present invention can be prepared via culture under adequate conditions using media which is conventionally used for culture of the lactic acid bacteria. In this case, a natural or synthetic medium can be used, as long as it contains a carbon source, a nitrogen source, mineral salts, and the like, and a lactic acid bacterium can be cultured efficiently therein. A person skilled in the art can adequately select a known medium suitable for a bacterial strain to be used. Examples of a carbon source that can be used herein include lactose, glucose, sucrose, fructose, galactose, and blackstrap molasses. Examples of a nitrogen source that can be used herein include organic nitrogen-containing substances such as casein hydrolysate, whey protein hydrolysate, and soy protein hydrolysate. In addition, examples of mineral salts that can be used herein include phosphate, sodium, potassium, and magnesium. Examples of media suitable for culture of lactic acid bacteria include MRS liquid medium, GAM medium, BL medium, Briggs Liver Broth, animal milk, skim milk, and milk-derived whey. Preferably, sterilized MRS medium can be used.

Furthermore, the CP2305s strain, or a cold-tolerant derivative strain from the CP2305 strain or the CP2305s strain according to the present invention may be cultured at 20°C to 50°C, preferably at 25°C to 42°C, and more preferably at about 37°C under anaerobic culture conditions. Temperature conditions can be adjusted using a thermostatic bath, a mantle heater, a jacket, or the like. The term "anaerobic conditions" as used herein refers to a low-oxygen environment in which bacteria can proliferate. For example, anaerobic conditions can be provided using an anaerobic chamber, an anaerobic box, or an airtight container or a bag containing a deoxidizer, or by simply sealing a culture container. A culture format may be any of static culture, shake culture, and tank culture, for example. The period of culture can be 3 hours to 100 hours or longer, and preferably about 10 hours to 50 hours. It may be preferable to maintain the pH of a medium at the beginning of culture between 3.5 and 8.0.

After culturing, living cells separated by a separation technique including filtration and centrifugation from culture media, or culture media containing living cells can be obtained. Also, these living cells or the culture media are subjected to known sterilization using an autoclave or the like, so that sterilized cells thereof can be obtained. In addition, treated product may be prepared from living cells or living cell-containing culture media and then subjected to sterilization, as necessary.

Treated products are obtained by subjecting cells to drying treatment, breaking or disruption treatment, or fermentation. Therefore, the treated product to be used in the present invention include dried cells, broken cells, disrupted cells, and fermented milk.

Dried cells can be obtained by subjecting cells separated from the above culture media, or cells subjected to sterilization as necessary, to drying treatment. Examples of drying treatment include drum dry, spray dry, vacuum dry, and lyophilization.

Broken cells or disrupted cells can be obtained by treating cells by disruption, grinding, enzymatic treatment, chemical treatment, lysis treatment or the like. Broken cells or disrupted cells substantially consist of all solid components and soluble components of broken or disrupted cells. For example, broken cells or disrupted cells can be obtained by directly subjecting broken or disrupted products to lyophilization. Breaking or disruption can be performed by a known technique such as physical disruption, enzymatic lysis treatment, chemical treatment, or the like. Physical disruption may be carried out using either a wet type or a dry type. Specifically, physical disruption may be carried out by agitation using, for example, homogenizer, ball mill, bead mill, Dyno-Mill, or satellite mill, or by using, for example, jet mill, French press, cell disruptor, or the like. Enzymatic lysis treatment can be performed by breaking the cell structure of a bacterium using an enzyme, such as lysozyme. Chemical treatment can be performed by breaking the cell structure of a bacterium using a surfactant, such as glycerin fatty acid ester or soybean phospholipid. Preferably, physical disruption may be performed.

A specific method for preparing disrupted cells involves disrupting cells, for example, treating a bacterial suspension in a known Dyno-Mill cell disruptor (e.g., Dyno-Mill disruptor (Shinmaru Enterprises Corporation (Japan, Osaka, Sakai))) using glass beads at a peripheral speed of 10,0 to 20.0 m/s (e.g., about 14.0 m/s) and a treating flow rate of 0.1 to 10 L/10 min (e.g., about 1 L/10 min) at a disruption tank temperature of 10°C to 30°C (e.g., about 15°C) 1 to 7 times (e.g., 3 to 5 times). Alternatively, cells can also be disrupted by treating a bacterial suspension using a known wet-type jet-mill cell disruptor (e.g., JN20 Nano Jet Pul (JOKOH (Japan, Kariagawa, Kawasaki))) at a discharge pressure of 50 to 1000 Mpa (e.g., 270 Mpa) at a treating flow rate of 50 to 1000 ml/min (e.g., 300 ml/min) 1 to 30 times (e.g., 10 times). Further, cells can also be disrupted by treating bacterial cell powder using a known dry-type satellite mill cell disruptor in the presence of any of different balls (e.g,, 10-mm zirconia balls, 5-mm zirconia balls, or 1-mm alumina balls) at 50 to 10,000 rpm (e.g., 240 rpm or 190 rpm) for 30 minutes to 20 hours (e.g., 5 hours). Cells may also be disrupted by treating several times bacterial cell powder using a known dry-type jet-mill cell disruptor (e.g., nano Jet-O-Mizer) at a feeding speed of 0.01 to 10000 g/min (e.g., 0.5 glmin) and a discharge pressure of 1 to 1,000 kg/cm² (e.g., 6 kg/cm²).

Fermented milk can be obtained by inoculating a bacterial strain to raw milk, fat-free milk powder, soybean milk, or the like, and then performing fermentation under fermentation conditions for lactic acid bacteria. The thus obtained fermentation product may be subjected to filtration, dilution, concentration, and the like, as necessary.

The above cells and treated product can be directly used, or, subjected to sterilization to obtain sterilized cells or product. Examples of sterilization include known treatments such as sterilization by high-energy ray irradiation, heat sterilization, pressure sterilization, and autoclave sterilization. A preferred sterilization method is autoclave sterilization. This method involves sterilizing living cells or culture media containing living cells within an autoclave (sterilizer using high-pressure steam) for about 10 minutes to 1 hour, for example, with pressurized water vapor at temperatures ranging from 110°C to 140°C, for example.

Because of the above properties, the CP2305 strain, the CP2305s strain, or a cold-tolerant derivative strain from the CP2305 strain or the CP2305s strain according to the present invention, or a treated product thereof are useful as an agent for alleviating excessive water secretion in the bowels, stress-induced bowel disorder, stress-induced diarrhea, irritable bowel syndrome, diarrhea-predominant irritable bowel syndrome, or, inflammatory bowel disease described below.

The term "stress-induced diarrhea" as used herein refers to symptoms involving excessive water secretion in the bowels under circumstances where a subject is exposed to stress so that an intracerebral stress hormone is secreted, that is, caused by stress. Stress-induced diarrhea may or may not involve an abdominal pain.

The term "stress-induced abdominal pain" as used herein refers to an abdominal pain that is induced by stress in the bowels under circumstances where a subject is exposed to stress so that an intracerebral stress hormone is secreted.

Therefore, subjects with "stress-induced diarrhea" and "stress-induced abdominal pain" as used herein are distinguished from patients with irritable bowel syndrome (IBS) in that particularly, stress-induced diarrhea and stress-induced abdominal pain are stress-induced symptoms triggered by stress to healthy individuals.

The term "stress-induced diarrhea" and "stress-induced abdominal pain" are together referred to herein as "stress-induced bowel disorder".

Therefore, the term "diarrheal symptoms of stress-induced bowel disorder" as used herein refers to the symptoms of stress-induced diarrhea.

The term "excessive water secretion in the bowels" as used herein refers to, regardless of whether or not caused by stress, excessive water secretion in the bowels.

The term "irritable bowel syndrome" as used herein refers to a disease or disorder characterized by the absence of inflammation or polyp or tumors (referred to as organic lesions) developed in/on intestinal mucosa and the advent of symptoms such as an abdominal pain or abnormal bowel movement (in particular, repeated diarrhea alternating with constipation) because of excessively-intense intestinal functions. In particular, diarrhea-predominant irritable bowel syndrome is characterized in that a sensitive reaction takes place during passage of intestinal content because of hyperesthesia of the large intestine, contraction encouraging bowel elimination is excessively induced, and diarrheal symptoms take place frequently, followed by chronic abdominal pain. Patients with diarrhea-predominant irritable bowel syndrome suffer successively from anxiety due to stress and thus have an increased incidence of developing complications (psychiatric disorders). The diagnostic criteria of diarrhea-predominant irritable bowel syndrome are as specified under the Rome III. Patients with diarrhea-predominant irritable bowel syndrome are patients with low QOL regardless of the presence or the absence of stress environment.

The term "inflammatory bowel disease" as used herein refers collectively to chronic diseases that cause cryptogenic inflammation mainly in the digestive tract, which comprises mainly two diseases: ulcerative colitis and Crohn's disease. Inflammatory bowel disease worsens due to stress and is accompanied by diarrheal symptoms.

The alleviating agent (or suppressing agent) according to the present invention contains an active ingredient the CP2305 strain, CP2305s strain, or a cold-tolerant derivative strain from the CP2305 strain or the CP2305s strain, a treated product thereof, or a mixture thereof, in an amount ranging from about 0.1% by weight to 100% by weight, and preferably from about 10% by weight to about 90% by weight per agent. However, examples of the amount are not limited thereto.

The alleviating agent (or suppressing agent) can contain, in addition to the above active ingredient, an excipient, a diluent or a carrier, and other additives as necessary. The form thereof may be either solid or liquid. Examples of a solid form include any forms such as powders, granules, tablets, and pellets. In addition, examples of a liquid form include suspensions, emulsions, and fermentation liquids (e.g., fermented milk).

Excipients, diluents, or carriers are substances that can be conventionally used in foods, animal feeds or pharmaceutical products described below. Other additives are not always required. However, when other additives are added, examples thereof include preservatives, pH adjusting agents, emulsifiers, antioxidants, and coloring agents as described below.

### <Food or animal feed>

Further described herein is a food additive or an animal feed additive containing living cells or sterilized cells of *Lactobacillus gasseri* CP2305 strain (FERM BP-11331), *Lactobacillus gasseri* CP2305s strain (NITE BP-1405), or a cold-tolerant derivative strain from the CP2305 strain or the CP2305s strain, a treated product thereof, or a mixture thereof, or, the alleviating agent (or suppressing agent).

The alleviating agent (or suppressing agent) containing an excipient, a diluent, or a carrier, and other additives, in addition to an active ingredient, can be used as a food additive or an animal feed additive. The form of these food additives or animal feed additives may be either solid or liquid. Examples of a solid form include any forms such as powders, granules, tablets, pellets, gels, and capsules. In addition, examples of a liquid form include suspensions, emulsions, and fermentation liquids (e.g., fermented milk). Excipients, diluents, or carriers are substances that can be conventionally used for foods or animal feeds. Examples of other additives include preservatives, pH adjusting agents, emulsifiers, coloring agents, supplements, (natural or artificial) sweeteners, seasonings, and flavors.

Each food additive or animal feed additive may contain the alleviating agent in an amount ranging from about 1% by weight to about 50% by weight, preferably from about 5% by weight to about 20% by weight, for example. However, examples of the content are not limited thereto.

The food additive or the animal feed additive is added to a food or an animal feed, and thus can be used for alleviating excessive water secretion in the bowels, stress-induced diarrhea, stress-induced bowel disorder, irritable bowel syndrome or inflammatory bowel disease in subjects.

Further described herein is a method for producing an animal feed, which comprises mixing an animal feed containing the alleviating agent (or suppressing agent), or, the alleviating agent (or suppressing agent) with animal feed ingredients, and thus producing an animal feed having an effect of alleviating excessive water secretion in the bowels, stress-induced diarrhea, stress-induced bowel disorder, irritable bowel syndrome, or inflammatory bowel disease in subjects.

Specifically, an animal feed can be produced by mixing an effective dose of the agent according to the present invention with feed ingredients for production of an animal feed, and then formulating the mixture into any dosage form, as necessary.

Further described herein is a method for producing a functional food or drink, which comprises mixing living cells or sterilized cells of *Lactobacillus gasseri* CP2305 strain (FERM BP-11331), *Lactobacillus gasseri* CP2305s strain (NITE BP-1405), or a cold-tolerant derivative strain from the CP2305 strain or the CP2305s strain, a treated product thereof, or a mixture thereof, or, a food or drink or a functional food or drink containing the alleviating agent, or, mixing the alleviating agent with a carrier, an excipient or a diluent for foods, and further mixing with food additives as necessary, and thus producing a functional food or drink having an effect of alleviating excessive water secretion in the bowels, stress-induced diarrhea, stress-induced bowel disorder, irritable bowel syndrome, or, inflammatory bowel disease in subjects.

For animal feeds or functional foods or drinks, the amount of the agent can be adequately determined by a person skilled in the art in consideration of the form of a feed or a food or drink and a required effect (that is, an effect of suppressing excessive water secretion in the bowels of a subject, and suppressing stress-induced bowel disorder, stress-induced diarrhea, and the like). The total amount of sterilized cells or living cells and/or treated product of the CP2305 strain, the CP2305s strain, or a cold-tolerant derivative strain from the CP2305 strain or the CP2305s strain in an animal feed or a functional food or drink corresponds to (but the examples thereof are not limited thereto) the viable count of the relevant bacterium ranging from 10⁵ to 10¹⁴cfu/g, preferably from 10⁶ to about 10¹³cfu/g, and more preferably from 10⁷ to 10¹²cfu/g, or, the amount of the cells ranging from, for example, 0.001% by weight to 10% by weight, preferably from 0.01% by weight to 5% by weight. The effective dose can be adequately determined by a person skilled in the art based on the relationship between the amount and the effect of alleviating diarrheal symptoms.

When the dosage form of an animal feed is solid, examples include any form such as powders, granules, tablets, and pellets. When the dosage form of the same is liquid, examples include suspensions and fermented milk.

When the dosage form of a functional food is solid, examples include any form such as powders, granules, tablets, pellets, gels, and capsules. When the dosage form of the same is liquid, examples include suspensions, emulsions, and fermented milk.

Among the above dosage forms, tablets, pellets, and capsules can be coated with an enteric coating substance, so as to prepare monolayered or multi-layered enteric-coated formulations, as necessary. Enteric coating substances are not dissolved at acidic pH (of gastric juice or the like), but dissolved at neutral pH, and examples thereof include hypromellose phthalate, shellac, zein, lactoferrin, hydroxypropylmethylcellulose phthalate, hydroxypropylmethylcellulose acetate succinate, carboxymethylethyl cellulose, and cellulose acetate phthalate.

Examples of excipients, diluents or carriers include, but are not limited to, water, milk-fermented solution, carbonated water, ethanol, polyvinyl alcohol, polyvinyl pyrrolidone, carboxyvinyl polymers, sodium alginate, water-soluble dextran, water-soluble dextrin, sodium carboxymethyl starch, carboxymethyl cellulose, starch, pectin, xanthan gum, gum Arabic, casein, gelatin, agar, glycerin, propylene glycol, polyethylene glycol, Vaseline, paraffin, stearyl alcohol, mannitol, sorbitol, and lactose.

For production of animal feeds or functional foods or drinks, various additives that are conventionally used in feeds or foods or drinks can be used. Examples of the additives include, but are not limited to, coloring agents (e.g., plant pigments and synthetic dyes), flavors (e.g., natural flavors), sweeteners (e.g., stevia and aspartame), preservatives (e.g., sodium acetate and sorbic acid), emulsifiers (e.g., sodium chondroitin sulfate and propylene glycol fatty acid ester), antioxidants (e.g., disodium EDTA and vitamin C), pH adjusting agents (e.g., citric acid), chemical seasonings (e.g., sodium inosinate and sodium glutamate), thickeners (e.g., xanthan gum), swelling agents (e.g., calcium carbonate), antifoaming agents (e.g., calcium phosphate), binders (e.g., sodium polyphosphate), and nutrition-enriching agents (e.g., calcium-enriching agents and vitamin A). Moreover, various nutrients, various vitamins, minerals, dietary fibers, acidulants, stabilizers, flavors, natural extracts, and the like can be added herein. These additives can be added in amounts so that the effects thereof are exhibited and can be adequately selected.

### <Pharmaceutical composition>

Further described herein is a pharmaceutical composition for preventing, alleviating or suppressing, or treating stress-induced bowel disorder, which contains living cells or sterilized cells of *Lactobacillus gasseri* CP2305 strain (FERM BP-11331), *Lactobacillus gasseri* CP2305s strain (NITE BP-1405), or a cold-tolerant derivative strain from the CP2305 strain or the CP2305 strain or the CP2305s strain, a treated product thereof, or a mixture thereof, as an active ingredient.

Alternatively, described herein is a pharmaceutical composition for preventing, alleviating or suppressing, or treating stress-induced bowel disorder in subjects, and particularly excessive water secretion and stress-induced diarrhea, which contains the alleviating agent (or suppressing agent) and a pharmaceutically acceptable carrier, excipient, or diluent.

In both pharmaceutical compositions, the active ingredient of the present invention has an effect of suppressing stress-induced bowel disorder, and particularly excessive water secretion and stress-induced diarrhea.

The CP2305 strain, the CP2305s strain, or a cold-tolerant derivative strain from the CP2305 strain or the CP2305 strain or the CP2305s strain (living cells or sterilized cells of these strains), or a treated product thereof have, as described above, an effect of inhibiting the stress-induced suppression of pelvic (parasympathetic) nerve; that is, bowel movements are maintained with its anti-stress functions at a normal level, and, an effect of suppressing the chloride channels of the large intestine, so as to suppress excessive water secretion in the bowels, thereby suppressing, alleviating, or improving stress-induced diarrhea. Stress triggers stress-sensitive intestinal disturbance, worsening the symptoms thereof. The CP2305 strain, the CP2305s strain, or a cold-tolerant derivative strain from the CP2305 strain or the CP2305s strain according to the present invention (living cells or sterilized cells of these strains), or a treated product thereof can suppress stress-induced bowel disorder, and particularly frequent diarrheal symptoms of stress-induced bowel disorder, and thus are effective for preventing, alleviating or suppressing, or treating stress-induced bowel disorder. This effect was confirmed to be higher than that of all commercially available formulations of *Lactobacillus gasseri* strain.

The pharmaceutical composition can be formulated into any dosage form, as long as it can exhibit the above effect. Examples thereof include tablets, capsules, granules, powders, dust formulations, syrups, dry syrups, liquids, suspensions, emulsions, and fermented milk agents. Further examples thereof include oral formulations and suppositories, and oral formulations are particularly preferred.

To the active ingredient of the present invention, additives such as pharmaceutically acceptable and conventionally used excipients, carriers, diluents, disintegrators, binders, wetting agents, stabilizers, buffers, lubricants, preservatives, surfactants, sweeteners, corrigents, aromatics, acidulants, and coloring agents may be added depending on dosage forms, and thus the pharmaceutical composition described herein can be produced using conventional methods. Examples of these substances are as described above.

The total amount of the CP2305 strain, the CP2305s strain, or a cold-tolerant derivative strain from the CP2305 strain or the CP2305s strain, and/or a treated product thereof, in the pharmaceutical composition, corresponds to, but is not limited to: the viable count of the relevant bacterium ranging from 10⁵ to 10¹²cfu/g, preferably from 10⁶ to about 10¹²cfu/g, and more preferably 10⁷ to 10¹²cfu/g, or; the amount of the cells ranging from, for example, 0.001% by weight to 10% by weight, and preferably from 0.01% by weight to 5% by weight. The effective dose thereof can be adequately determined by a person skilled in the art based on the relationship between the amount and the effect of alleviating diarrheal symptoms.

A preferred mode of administration may be oral administration or administration of a suppository.

The pharmaceutical composition described herein may be effective for preventing excessive water secretion in the bowels, stress-induced bowel disorder, irritable bowel syndrome or inflammatory bowel disease, and preventing, alleviating or treating the worsening of stress-triggered diarrheal symptoms.

### Examples

The present invention will hereinafter be described in greater detail with reference to the following examples, although the scope of the present invention is not limited thereto.

### Example 1

### <Breeding of cold-tolerant strains>

*Lactobacillus gasseri* CP2305 strain (bacterial count ranging from 10³ to 10⁴) was inoculated into 10 ml of a culture medium (medium supplemented with food additives) in a 15-ml polypropylene test tube with an inside diameter of 15 mm and then cultured at 37°C for 19 hours.

After 19 hours of culture, the 15-ml polypropylene test tube with an inside diameter of 15 mm was inserted (on ice) into a sufficient amount of ice, so that the level of ice was higher than the liquid level within the test tube for rapid cooling, and then kept for 5 hours. This culture and cooling were repeated 80 times, thereby breeding a highly cold-tolerant strain.

The term "on ice" as used herein refers to a state in which a 15-ml polypropylene test tube with an inside diameter of 15 mm containing 10 ml of a culture medium is inserted into a sufficient amount of ice so that the level of ice is higher than the liquid level within the test tube for cooling.

In addition, the composition of the "medium supplemented with food additives" as used herein are as follows (Table 1).

**[Table 1]**

| Composition of medium supplemented with food additives | |
|---|---|
| Composition | Content (g/l) |
| Casein Pepton PLUS (Organotechnie) | 10 |
| Bacterio-N-KS (Maruha Nichiro Foods, Inc.) | 10 |
| HF-55 (Gun Ei Chemical industry) | 30 |
| Sodium acetate (crystal) (NIPPON GOHSEI) (CH₃COONa·3H₂O) | 5 |
| Dipotassium hydrogen phosphate (anhydrous) (Taihei Chemical Industrial Co. Ltd.) | 4.5 |
| Magnesium sulfate (heptahydrate) (Tomita Pharmaceutical Co., Ltd.) | 1 |
| Yeast extract (Ohly, Yeast=KAT) | 5 |
| Sunsoft Q-17S (Talyo Kaaaku Co., Ltd.) | 1 |

### <Evaluation of cold-tolerant strains>

Regarding evaluation of the cold-tolerant strains according to the present invention, the cold-tolerant strains were evaluated for viability based on the percentage of living cells (also herein referred to as "survival rate") before and after exposure to low temperature, as in the following formula. Specifically, a strain (bacterial count ranging from 10³ to 10⁴) to be evaluated was inoculated into 10 ml of a culture medium (medium supplemented with food additives) in a 15-ml polypropylene test tube with an inside diameter of 15 mm, and then cultured at 37°C for 19 hours. After 19 hours of culture, the 15-ml polypropylene test tube with an inside diameter of 15 mm was inserted (on ice) into a sufficient amount of ice for rapid cooling, so that the level of ice is higher than the liquid level within the test tube, and then kept on ice for 5 hours. The number of living cells before rapid cooling (before exposure to low temperature) was compared with the number of living cells after kept on ice for 5 hours (after exposure to low temperature), and thus the percentage of living cells was evaluated with the following formula.

A preferred strain exhibits the percentage of living cells (see the following formula) of at least 60%, preferably 65% or higher, further preferably, 70% or higher, 75% or higher, 80% or higher, 85% or higher, 90% or higher, and 95% or higher.

Percentage of living cells (%) = number of living cells after exposure to low temperature/number of living cells before exposure to low temperature × 100

A method for counting the number of living cells is as follows. An MRS agar medium or a medium of plate count agar with BCP was coated with the culture medium, cells were cultured microaerophillically at 37°C for 48 hours, colonies were counted, and thus the number of living cells were measured and evaluated.

As a result of the above breeding and evaluation, several cold-tolerant strains, including the CP2305s strain exhibiting the percentages of living cells of 60% or higher, were obtained (Fig. 1).

### Example 2

### <Method for producing cold-tolerant strains>

In addition, a method for producing cold-tolerant strains is as follows. For example, the survival rate of the CP2305 strain tends to decrease at 10°C or lower. Therefore, a method for producing cold-tolerant strains is performed by culturing a test strain in a culture medium (e.g., medium supplemented with food additives) at an appropriate culture temperature (e.g,, about 37°C) for an appropriate period of culture (e.g., about 19 hours), and then exposing the culture medium to a temperature of 10°C or lower, preferably 7°C or lower, more preferably 5°C or lower, further preferably 3°C or lower, 2°C or lower, or 1°C or lower for an appropriate period of time (e.g., 5 hours), and, if necessary, repeating the culture and exposure to low temperatures, and thus producing a strain having resistance to a target low temperature.

### Example 3

SD male rats (4-week-old, CHARLES RiVER LABORATORIES JAPAN, INC.) were raised for 1 week under a light-dark cycle of every 12 hours (lighting for 12 hours from eight), and fed ad libitum with a feed containing test cells for 3 weeks as described below,
a) CRF-1 powder feed (Oriental Yeast Co., ltd.) was used.
b) Lyophilized powders of test cells (bacterial count 2.0 × 10¹⁰) was mixed with 25 g of the above feed (CRF-1) (to 25 g of CRF-1 feed, 0.25 g of lyophilized powers was added); thereby preparing a feed containing test cells.
c) Rats were fed *ad litibum* with the feed containing test cells for 3 weeks,
d) The amount of cells taken in this test was assumed to be about 2.0 × 10¹⁰ cells/day.
e) 19 rats of each group were fed.

After 3 weeks, a CRF (Corticotropin Releasing Factor, AnaSpec, Inc., U.S.A.) solution was intraperitoneally administered at 125 µg/kg, so as to induce stress-induced diarrhea. Fecal status observed for 100 minutes after administration of CRF was evaluated based on the diarrhea score described below, and thus the suppression of stress-induced diarrhea by the test cells was evaluated.

The diarrhea score is as defined below.
(a) Feces are collected 10 times at intervals of 10 minutes for 100 minutes after administration of CRF.
(b) The weight of feces collected each time is measured with the unit of gram (the weight of feces collected each time (value "i")). For example, when the fecal weight in one measurement is 0.4 g, value "i" = 0.4.
(c) Feces collected each time are quantified as the fecal score of each measurement according to the fecal score criteria shown in Fig. 5 based on the water-content state and the shape of the feces (fecal score of each measurement (value "ii"))
(d) Fecal weight (value "i") of each measurement × fecal score (value "ii") of each measurement is obtained.
(e) The sum total of the ten results of "fecal weight (value "i") × fecal score (value "ii") (in each measurement)" is defined as "diarrhea score".
(f) Therefore, fecal status (also including placebo (CRF-1 alone) for 100 minutes after administration of CRF.) is evaluated (evaluated by Dunnet's multiple comparison).

As a result, living cells and sterilized cells of *Lactobacillus gasseri* CP2305 strain according to the present invention significantly suppressed stress-induced diarrhea compared with the placebo (CRF-1 alone) (Fig. 2).

### Example 4

The following effect was confirmed based on the above experiment and the above-defined diarrhea score.

As a result, *Lactobacillus gasseri* CP2305s strain (sterilized cells) significantly suppressed stress-induced diarrhea compared with the placebo (CRF-1 alone) (Fig. 2). The CP2305s strain had the effect of suppressing diarrhea to a degree equivalent to that of the CP2305 strain (living cells and sterilized cells).

The results of Example 3 above demonstrated that a lactic acid bacterium with a diarrhea score (in Example 3) of 11 or less was obtained. The diarrhea score is preferably 10 or less and further preferably 9 or less.

Furthermore, the results of Example 3 demonstrated that a lactic acid bacterium exhibiting the percentage of decrease in diarrhea score accounting for 90% or lower with respect to that of a placebo was obtained. The percentage of decrease in diarrhea score is preferably 85% or lower, and more preferably 80% or lower (percentage of decrease in diarrhea score = diarrhea score of strain to be evaluated/diarrhea score of placebo × 100).

### Example 5

The result for the cells of deposited strain A (L. *gassei*) as a subject to be compared was obtained by administering the cells in the same bacterial count as that of the CP2305 strain. In addition, the result for commercial probiotic formulation B (*L*. *acidphilus*) was obtained by administering the formulation so that the dose thereof to be taken by a rat per day was the same dose per unit body weight with respect to the dose to be taken by a human per day (Fig. 6). The result demonstrated that the CP2305 strain has an effect of suppressing stress-induced diarrhea to a degree higher than that of commercially available living cell formulations conventionally thought to have an effect of suppressing diarrhea. Since the CP2305s strain also has an effect of suppressing stress-induced diarrhea similar to that of the CP2305 strain, the superiority of the CP2305s strain was demonstrated.

### Example 6

Furthermore, dissection was performed 4 hours after administration of CRF, total RNA was extracted from the colon tissue using ISOGEN (Nippon Gene) and RNeasy Mini Kit (Qiagen,U.S.A.), and then gene expression was comprehensively analyzed with Rat whole genome ver. 3 (G4847B; Agilent, U.S.A.). As a result, it was confirmed that whereas the expression of chloride channel-related genes was increased after administration of CRF in the placebo group, the expression of the same was suppressed in the group to which the CP2305 strain had been administered (Fig. 3). Accordingly, the CP2305 strain was considered to suppress chloride channels, so as to control the transfer of water. Moreover, the CP2305s strain having a similar effect of suppressing diarrhea was considered to suppress chloride channels, so as to control the transfer of water.

### Example 7

SD male rats (4-week-old, Kiwa Laboratory Animals Co., Ltd.) were raised for 1 week under a light-dark cycle of every 12 hours (12 hours of lighting from eight), and then fed ad libitum with a CP2305 strain-containing feed or a control powder feed containing no such strain for 3 weeks. On the day of the experiment, the rats were fasted for 3 hours, urethane-anesthetized, a cannula for intravenous administration was inserted into the cervical vein, pelvic (parasympathetic) nerve innervating the lower part of the large intestine was lifted using a silver electrode, and then the electrical activity was measured. At a period of time when the measurement values were stable, a CRF solution (1 µg/kg) was administered via cervical vein, and then changes in pelvic nerve (electrical) activity (PNA) were measured electrophysiologically. Data were analyzed based on the mean value of firing frequencies per 5 seconds every 5 minutes, and each result is represented by percentage when the value (value at 0 minute) immediately before administration via cervical vein is expressed as 100%. As a result, it was confirmed that the administration of a stress hormone (CRF) suppresses the activity of pelvic (parasympathetic) nerve that innervatesrat large intestine. Furthermore, it was confirmed that the administration of the CP2305 strain inhibits the stress-induced suppression of pelvic (parasympathetic) nerve (Fig. 4). Therefore, the administration of the CP2305 strain is considered to maintain bowel functions under stress at a normal level. Moreover, the CP2305s strain having a similar effect of suppressing diarrhea is also considered to have a similar effect.

### Example 8

SD rats (body weights ranging from 380 × to 400 g, Japan SLC Inc.) were anesthetized, decapitated, and then sacrificed by bleeding. The distal colon was excised therefrom in accordance with anatomical standards, circular muscle and longitudinal muscle layers were removed under a stereo microscope, and then specimens of submucous plexus-containing mucous membrane-submucous plexus were prepared. All specimens were mounted on Ussing chambers (CHM2, WPI, FL, U.S.A.) each with a measurement area of 0.64 cm², and the potential difference and short-circuit current (I_{SC}) generated between the mucous membrane side and the serous membrane side of each specimen was measured. A 10-mV command pulse was applied to the tissue for 3 seconds per minute, and then transmembrane conductance (Gt) was calculated based on short-circuit current change. When basel I_{SC} and Gt became stable, electrical stimulation (25V, duration of 0.5ms, 5Hz, 120s) was applied to the submucosal nerve, so as to confirm the tissue status. Subsequently, test lactic acid bacterial cells or a diluted solution (500 µg/ml) of fermented milk was administered to the mucous membrane side, changes in I_{SC} and Gt were measured, and thus the effects of test substances on Cl⁻/HCO₃⁻ secretion induced by nerve stimulation were evaluated.

As a result of evaluating the effects of the sterilized fermented milk of various lactic acid bacteria, it was suggested that the sterilized fermented milk of the CP2305 strain suppressed ion secretion more strongly than that of the known deposited strain A (*L*. *gasseri*) and 5 other strains (B to F), and also controlled water secretion, and thus contributed to the suppression of diarrhea (Fig. 7).

### Example 9

Moreover, for evaluation of the effects of cells alone on nerve stimulation-induced ion transport in rat large intestine (distal colon), sterilized cells of various lactic acid bacteria were prepared, and then administered. As a result, it was suggested that sterilized cells of the CP2305 strain strongly suppressed ion secretion to a more significant extent than sterilized cells of the known deposited strain A (*L*. *gasseri*) and sterilized cells of *L. acidophilus,* and thus strongly contributed to the suppression of diarrhea (Fig. 8).

Furthermore, the effects of the administration of living cells, fermented milk of living cells, sterilized cells, and the sterilized fermented milk of the CP2305 strain on the nerve stimulation-induced ion transport in rat large intestine (distal colon) were examined. As a result, it was suggested that all forms thereof, and particularly sterilized cells of the CP2305 strain strongly contributed to the suppression of diarrhea (Fig. 9).

### Industrial Applicability

*Lactobacillus gasseri* CP2305 strain (FERM BP-11331), *Lactobacillus* gasseri CP2305s strain (NITE BP-1405), or a derivative strain (for example, a cold-tolerant derivative strain) derived from the CP2305 strain or the CP2305s strain; a treated product thereof, or a mixture thereof according to the present invention can inhibit the stress-induced suppression of pelvic (parasympathetic) neural activity and maintain intestinal motility at a normal level, as well as can suppress, alleviate, or improve stress-induced bowel disorder of subjects, and particularly the stress-induced diarrheal symptoms of the disorder.

## Claims

1. *Lactobacillus gasseri* CP2305 strain (FERM BP-11331), a cold-tolerant derivative strain derived from *Lactobacillus gasseri* CP2305 strain (FERM BP-11331), a treated product thereof, or a mixture thereof for use in suppressing stress-induced diarrhea in a healthy subject, wherein the treated product thereof is dried cells, broken cells, disrupted cells, or in the form of fermented milk.

2. *Lactobacillus gasseri* CP2305s strain (NITE BP-1405) or a cold-tolerant derivative strain derived from *Lactobacillus gasseri* CP2305 strain (FERM BP-11331) or the CP2305s strain (NITE BP-1405), a treated product thereof, or a mixture thereof for use in alleviating excessive water secretion in the bowels, stress-induced diarrhea, stress-induced bowel disorder, irritable bowel syndrome, or inflammatory bowel disease, wherein the treated product thereof is dried cells, broken cells, disrupted cells, or in the form of fermented milk.

3. The strain, treated product thereof, or mixture thereof for use according to claim 1 or claim 2, wherein the cold-tolerant strain is able to survive exposure to a temperature of 10°C or lower.

4. The strain, treated product thereof, or mixture thereof for use according to claims 1 to 3, wherein the cells of the CP2305 strain, the CP2305s strain, or the derivative strain derived from *Lactobacillus gasseri* CP2305 strain (FERM BP-11331) or the CP2305s strain (NITE BP-1405) are living cells or sterilized cells.

5. The strain, treated product thereof, or mixture thereof for use according to any one of claims 1 to 4, which is further for inhibiting the stress-induced suppression of pelvic (parasympathetic) neural activity.

6. The strain, treated product thereof, or mixture thereof for use according to any one of claims 1 to 5, which is further for suppressing water secretion by suppressing the chloride channels of the large intestine.

7. The strain, treated product thereof, or mixture thereof for use according to any one of claims 1 to 6, which is an agent for use in improving stress-induced diarrhea.

8. A food or drink, preferably a functional food or drink comprising:
a) living cells or sterilized cells of a *Lactobacillus gasseri* strain according to claim 1, a treated product thereof, or a mixture thereof, for use according to claim 1; or
b) living cells or sterilized cells of a *Lactobacillus gasseri* strain according to claim 2, a treated product thereof, or a mixture thereof, for use according to claim 2.

9. A lactic acid bacterium, which is *Lactobacillus gasseri* CP2305s strain (NITE BP-1405), or a cold-tolerant derivative strain derived from *Lactobacillus gasseri* CP2305 strain (FERM BP-11331) or the CP2305s strain.

10. The lactic acid bacterium according to claim 9, wherein the cold-tolerant derivative strain is able to survive exposure to a temperature of 10°C or lower.

## Patentansprüche

1. *Lactobacillus-gasseri-*CP2305-Stamm (FERM BP-11331), kältetoleranter Derivatstamm, abgeleitet von dem *Lactobacillus-gasseri-*CP2305-Stamm (FERM BP- 11331), behandeltes Produkt daraus oder Gemisch davon zur Verwendung bei der Unterdrückung stressbedingten Durchfalls bei einem gesunden Subjekt, wobei das behandelte Produkt davon getrocknete Zellen, zerstörte Zellen oder aufgeschlossene Zellen ist oder es in Form von fermentierter Milch vorliegt.

2. *Lactobacillus-gasseri-*CP2305s-Stamm (NITE BP-1405) oder kältetoleranter Derivatstamm, abgeleitet von dem *Lactobacillus-gasseri-*CP2305-Stamm (FERM BP-11331) oder dem CP2305s-Stamm (NITE BP-1405), behandeltes Produkt daraus oder Gemisch davon zur Verwendung bei der Linderung von übermäßiger Wassersekretion im Darm, stressbedingtem Durchfall, stressbedingter Darmerkrankung, Reizdarmsyndrom oder entzündlicher Darmerkrankung, wobei das behandelte Produkt davon getrocknete Zellen, zerstörte Zellen oder aufgeschlossene Zellen ist oder es in Form von fermentierter Milch vorliegt.

3. Stamm, behandeltes Produkt daraus oder Gemisch davon zur Verwendung nach Anspruch 1 oder Anspruch 2, wobei der kältetolerante Stamm fähig ist, Exposition gegenüber einer Temperatur von 10 °C oder weniger zu überleben.

4. Stamm, behandeltes Produkt daraus oder Gemisch davon zur Verwendung nach Anspruch 1 bis 3, wobei es sich bei den Zellen des CP2305-Stammes, des CP2305s-Stammes oder des Derivatstammes, abgeleitet von dem *Lactobacillus-*gasseri-CP2305-Stamm (FERM BP-11331) oder dem CP2305s-Stamm (NITE BP-1405), um lebende Zellen oder sterilisierte Zellen handelt.

5. Stamm, behandeltes Produkt daraus oder Gemisch davon zur Verwendung nach einem der Ansprüche 1 bis 4, welcher/s ferner zum Hemmen der stressbedingten Unterdrückung von (parasympathischer) Beckennervenaktivität dient.

6. Stamm, behandeltes Produkt daraus oder Gemisch davon zur Verwendung nach einem der Ansprüche 1 bis 5, welcher/s ferner zur Unterdrückung von Wassersekretion durch Unterdrückung der Chloridkanäle des Dickdarms dient.

7. Stamm, behandeltes Produkt daraus oder Gemisch davon zur Verwendung nach einem der Ansprüche 1 bis 6, wobei es sich um ein Mittel zur Verwendung bei der Verbesserung stressbedingten Durchfalls handelt.

8. Lebensmittel oder Getränk, bevorzugt funktionelles Lebensmittel oder Getränk, umfassend:
a) lebende Zellen oder sterilisierte Zellen eines *Lactobacillus-gasseri-Stammes* nach Anspruch 1, ein behandeltes Produkt daraus oder Gemisch davon, zur Verwendung nach Anspruch 1; oder
b) lebende Zellen oder sterilisierte Zellen eines *Lactobacillus-gasseri-Stammes* nach Anspruch 2, ein behandeltes Produkt daraus oder Gemisch davon, zur Verwendung nach Anspruch 2.

9. Milchsäurebakterium, bei dem es sich um den *Lactobacillus-gasseri-*CP2305s-Stamm (NITE BP-1405) oder einen kältetoleranten Derivatstamm, abgeleitet von dem *Lactojbacillus-gasseri-*CP2305-Stamm (FERM BP-11331) oder dem CP2305s-Stamm, handelt.

10. Milchsäurebakterium nach Anspruch 9, wobei der kältetolerante Derivatstamm in der Lage ist, Exposition gegenüber einer Temperatur von 10 °C oder weniger zu überleben.

## Revendications

1. Souche de *Lactobacillus gasseri* CP2305 (FERM BP-11331), souche dérivée tolérante au froid issue de *Lactobacillus gasseri* CP2305 (FERM BP-11331), produit traité correspondant, ou mélange correspondant pour une utilisation dans la suppression d'une diarrhée induite par le stress chez un sujet sain, le produit traité correspondant étant des cellules séchées, des cellules cassées, des cellules perturbées ou sous la forme d'un lait fermenté.

2. Souche de *Lactobacillus gasseri* CP2305s (NITE BP-1405), souche dérivée tolérante au froid issue de *Lactobacillus gasseri* CP2305 (FERM BP-11331) ou de la souche CP2305s (NITE BP-1405), produit traité correspondant, ou mélange correspondant pour une utilisation dans le soulagement d'une sécrétion d'eau excessive dans les intestins, d'une diarrhée induite par le stress, d'un trouble intestinal induit par le stress, d'un syndrome de l'intestin irritable ou d'une maladie intestinale inflammatoire, le produit traité correspondant étant des cellules séchées, des cellules cassées, des cellules perturbées ou sous la forme d'un lait fermenté.

3. Souche, produit traité correspondant ou mélange correspondant pour une utilisation selon la revendication 1 ou la revendication 2, la souche tolérante au froid étant capable de survivre à une exposition à une température de 10 °C ou moins.

4. Souche, produit traité correspondant ou mélange correspondant pour une utilisation selon les revendications 1 à 3, les cellules de la souche CP2305, la souche CP2305s ou la souche dérivée issue de *Lactobacillus gasseri* CP2305 (FERM BP-11331) ou la souche CP2305s (NITE BP-1405) étant des cellules vivantes ou des cellules stérilisées.

5. Souche, produit traité correspondant ou mélange correspondant pour une utilisation selon l'une quelconque des revendications 1 à 4, qui est en outre pour l'inhibition de la suppression induite par le stress de l'activité neuronale pelvienne (parasympatique).

6. Souche, produit traité correspondant au mélange correspondant pour une utilisation selon l'une quelconque des revendications 1 à 5, qui est en outre pour la suppression de la sécrétion d'eau en supprimant les canaux chlorure du gros intestin.

7. Souche, produit traité correspondant ou mélange correspondant pour une utilisation selon l'une quelconque des revendications 1 à 6, qui est un agent pour une utilisation dans l'amélioration de la diarrhée induite par le stress.

8. Produit alimentaire ou boisson, préférablement produit alimentaire ou boisson fonctionnel(le) comprenant :
a) des cellules vivantes ou des cellules stérilisées d'une souche de *Lactobacillus gasseri* selon la revendication 1, un produit traité correspondant ou un mélange correspondant, pour une utilisation selon la revendication 1 ; ou
b) des cellules vivantes ou des cellules stérilisées d'une souche de *Lactobacillus gasseri* selon la revendication 2, un produit traité correspondant ou un mélange correspondant, pour une utilisation selon la revendication 2.

9. Bactérie d'acide lactique qui est une souche de *Lactobacillus gasseri* CP2305s (NITE BP-1405) ou une souche dérivée tolérante au froid issue de *Lactobacillus gasseri* CP2305 (FERM BP-11331) ou de la souche CP2305s.

10. Bactérie d'acide lactique selon la revendication 9, la souche dérivée tolérante au froid étant capable de survivre à une exposition à une température de 10 °C ou moins.
